# EUROPÄISCHE PATENTANMELDUNG

(11) **EP 1 182 212 A2**
(43) Veröffentlichungstag der Anmeldung: **27.02.2002**
(21) Anmeldenummer: 01116616.2
(22) Anmeldetag: 12.07.2001
(51) Int. Cl.: C07K 14/72, C12Q 1/68, C07K 16/28, C12N 15/63

(54) **Ultraspiracle (USP)-Protein von Heliothis virescens**

(30) Priorität: 25.07.2000 DE 10036469
(71) Anmelder: BAYER AG, 51368 Leverkusen (DE)
(72) Erfinder: Zitzmann, Werner, Dr., 51381 Leverkusen (DE); Franken, Eva-Maria, Dr., 42799 Leichlingen (DE); Janssen, Martina, Dr., 53639 Königswinter (DE); Schulte, Thomas, Dr., 51061 Köln (DE)

(57) **Zusammenfassung**

Die Erfindung betrifft Nukleinsäuren, die für Polypeptide mit der biologischen Aktivität des Ultraspiracle-Proteins kodieren, sowie solche Polypeptide per se. Weiterhin betrifft die Erfindung Verfahren zum Auffinden insektizider Wirkstoffe, sowie zur kontrollierten Expression von Zielgenen (Gene Switch).

SEQUENZPROTOKOLL

<110> Bayer Aktiengesellschaft

<120> Ultraspiracle (USP)-Protein von Heliothis virescens

<130> Le A 34 771

<140>
<141>

<150> DE 100 36 469.1
<151> 2000-07-25

<160> 2

<170> PatentIn Ver. 2.1

<210> 1
<211> 1398
<212> DNA
<213> Heliothis virescens

<220>
<221> CDS
<222> (1)..(1398)

<400> 1
atg tcc gtg gcg aag aaa gac aag ccg aca atg tcg gtg aca gca ctt 48
Met Ser Val Ala Lys Lys Asp Lys Pro Thr Met Ser Val Thr Ala Leu
1               5                   10                  15

atc aac tgg gct cga ccc ttg ccg ccg ggc caa cag cag cag ccg atg 96
Ile Asn Trp Ala Arg Pro Leu Pro Pro Gly Gln Gln Gln Gln Pro Met
            20                  25                  30

acg cct acg tcg ccc gga aac atg ctt caa ccg atg gct acg ccg tct 144
Thr Pro Thr Ser Pro Gly Asn Met Leu Gln Pro Met Ala Thr Pro Ser
        35                  40                  45

aac tta ccg act gtc gac tgc tca ctc gat att caa tgg cta aac ttg 192
Asn Leu Pro Thr Val Asp Cys Ser Leu Asp Ile Gln Trp Leu Asn Leu
    50                  55                  60

gag gga ggt ttt atg tcg ccg atg tca ccg ccg gag atg aag cca gac 240
Glu Gly Gly Phe Met Ser Pro Met Ser Pro Pro Glu Met Lys Pro Asp
65                  70                  75                  80

acg gcg atg cta gac ggc ctg cga gac gac tcc acc cca ccc cca gct 288
Thr Ala Met Leu Asp Gly Leu Arg Asp Asp Ser Thr Pro Pro Pro Ala
                85                  90                  95

ttc aag aac tac ccc ccg aac cat ccc cta agt ggt tct aag cac ctc 336
Phe Lys Asn Tyr Pro Pro Asn His Pro Leu Ser Gly Ser Lys His Leu
            100                 105                 110

tgt tct ata tgt gga gat aga gcg tcg ggg aaa cat tat gga gta tac 384
Cys Ser Ile Cys Gly Asp Arg Ala Ser Gly Lys His Tyr Gly Val Tyr
        115                 120                 125

agt tgt gaa ggt tgc aaa ggt ttc ttc aaa agg acg gta aga aaa gac 432
Ser Cys Glu Gly Cys Lys Gly Phe Phe Lys Arg Thr Val Arg Lys Asp
    130                 135                 140

tta acg tac gca tgc cgc gaa gaa cgt aac tgc atc ata gac aaa cgc 480
Leu Thr Tyr Ala Cys Arg Glu Glu Arg Asn Cys Ile Ile Asp Lys Arg
145                 150                 155                 160

cag agg aac aga tgc cag tac tgt agg tac cag aaa tgt ctc gcg tgc 528
Gln Arg Asn Arg Cys Gln Tyr Cys Arg Tyr Gln Lys Cys Leu Ala Cys
                165                 170                 175

ggc atg aag agg gaa gcg gtg cag gag gag agg cag agg gcc gcc aga 576
Gly Met Lys Arg Glu Ala Val Gln Glu Glu Arg Gln Arg Ala Ala Arg
            180                 185                 190

ggt acg gag gat gca cat ccg agc agc tcg gtg cag gta cag gag tta 624
Gly Thr Glu Asp Ala His Pro Ser Ser Ser Val Gln Val Gln Glu Leu
        195                 200                 205

tca atc gag cgg ttg ctg gag atg gag tca ctg gta gct gac ccc agc 672
Ser Ile Glu Arg Leu Leu Glu Met Glu Ser Leu Val Ala Asp Pro Ser
    210                 215                 220

gaa gag ttc cag ttc ctt cgt gtg gga ccc gac agt aat gtg ccg cct 720
Glu Glu Phe Gln Phe Leu Arg Val Gly Pro Asp Ser Asn Val Pro Pro
225                 230                 235                 240

aag ttc cgc gcc cct gtc tcc agc ctt tgt caa ata ggc aac aaa caa 768
Lys Phe Arg Ala Pro Val Ser Ser Leu Cys Gln Ile Gly Asn Lys Gln
                245                 250                 255

ata gcg gcg cta gtg gtg tgg gcg cgc gac atc ccg cac ttc agc cag 816
Ile Ala Ala Leu Val Val Trp Ala Arg Asp Ile Pro His Phe Ser Gln
            260                 265                 270

ctt gag atg gaa gac cag atc ctg ctc atc aaa ggc tcc tgg aac gaa    864
Leu Glu Met Glu Asp Gln Ile Leu Leu Ile Lys Gly Ser Trp Asn Glu
        275                 280                 285

ctg ctg ctc ttc gcc att gcg tgg cgg tct atg gag ttc ctg aca gaa    912
Leu Leu Leu Phe Ala Ile Ala Trp Arg Ser Met Glu Phe Leu Thr Glu
    290                 295                 300

gag cga gac ggc gtg gac ggc act ggg aac aga acc aca tcg ccg cca    960
Glu Arg Asp Gly Val Asp Gly Thr Gly Asn Arg Thr Thr Ser Pro Pro
305                 310                 315                 320

caa ctt atg tgt ctc atg cct ggc atg acg ctg cac cgc aac tca gcg    1008
Gln Leu Met Cys Leu Met Pro Gly Met Thr Leu His Arg Asn Ser Ala
                325                 330                 335

ctg cag gcg ggc gtg ggg cag atc ttc gac cgc gtg ctg tcg gag ctg    1056
Leu Gln Ala Gly Val Gly Gln Ile Phe Asp Arg Val Leu Ser Glu Leu
            340                 345                 350

tcg ctg aag atg cgc acc ctg cgc gtc gac cag gcc gag tac gtc gcg    1104
Ser Leu Lys Met Arg Thr Leu Arg Val Asp Gln Ala Glu Tyr Val Ala
        355                 360                 365

ctc aag gcc atc ata ctg ctc aac cca gat gtg aag gga ctg aaa aac    1152
Leu Lys Ala Ile Ile Leu Leu Asn Pro Asp Val Lys Gly Leu Lys Asn
    370                 375                 380

agg caa gaa gtg gaa gtt tta cga gaa aag atg ttc ctg tgc ctg gac    1200
Arg Gln Glu Val Glu Val Leu Arg Glu Lys Met Phe Leu Cys Leu Asp
385                 390                 395                 400

gag tac tgc cgc cgc tcg cgc agt tcg gag gag ggt cgg ttc gcg gcg    1248
Glu Tyr Cys Arg Arg Ser Arg Ser Ser Glu Glu Gly Arg Phe Ala Ala
                405                 410                 415

ctg ctg ctg cgc ctg ccc gcg tta cgt tcc att tca ctc aag agc ttc    1296
Leu Leu Leu Arg Leu Pro Ala Leu Arg Ser Ile Ser Leu Lys Ser Phe
            420                 425                 430

gag cac ctg ttc ttc ttc cac ctg gtg gcc gac acc agc atc gcc ggc    1344
Glu His Leu Phe Phe Phe His Leu Val Ala Asp Thr Ser Ile Ala Gly
        435                 440                 445

tac atc cgc gac gcg ctg cgc aac cac gcg ccg ccc atc gac acc aac    1392
Tyr Ile Arg Asp Ala Leu Arg Asn His Ala Pro Pro Ile Asp Thr Asn
    450                 455                 460

atg atg                                                          1398
Met Met
465

<210> 2
<211> 466
<212> PRT
<213> Heliothis virescens

<400> 2
Met Ser Val Ala Lys Lys Asp Lys Pro Thr Met Ser Val Thr Ala Leu
1               5                   10                  15

Ile Asn Trp Ala Arg Pro Leu Pro Pro Gly Gln Gln Gln Gln Pro Met
            20                  25                  30

Thr Pro Thr Ser Pro Gly Asn Met Leu Gln Pro Met Ala Thr Pro Ser
        35                  40                  45

Asn Leu Pro Thr Val Asp Cys Ser Leu Asp Ile Gln Trp Leu Asn Leu
    50                  55                  60

Glu Gly Gly Phe Met Ser Pro Met Ser Pro Pro Glu Met Lys Pro Asp
65                  70                  75                  80

Thr Ala Met Leu Asp Gly Leu Arg Asp Asp Ser Thr Pro Pro Pro Ala
                85                  90                  95

Phe Lys Asn Tyr Pro Pro Asn His Pro Leu Ser Gly Ser Lys His Leu
            100                 105                 110

Cys Ser Ile Cys Gly Asp Arg Ala Ser Gly Lys His Tyr Gly Val Tyr
        115                 120                 125

Ser Cys Glu Gly Cys Lys Gly Phe Phe Lys Arg Thr Val Arg Lys Asp
    130                 135                 140

Leu Thr Tyr Ala Cys Arg Glu Glu Arg Asn Cys Ile Ile Asp Lys Arg
145                 150                 155                 160

Gln Arg Asn Arg Cys Gln Tyr Cys Arg Tyr Gln Lys Cys Leu Ala Cys
                165                 170                 175

Gly Met Lys Arg Glu Ala Val Gln Glu Glu Arg Gln Arg Ala Ala Arg
            180                 185                 190

Gly Thr Glu Asp Ala His Pro Ser Ser Ser Val Gln Val Gln Glu Leu

195                 200                 205

Ser Ile Glu Arg Leu Leu Glu Met Glu Ser Leu Val Ala Asp Pro Ser
    210                 215                 220

Glu Glu Phe Gln Phe Leu Arg Val Gly Pro Asp Ser Asn Val Pro Pro
225                 230                 235                 240

Lys Phe Arg Ala Pro Val Ser Ser Leu Cys Gln Ile Gly Asn Lys Gln
                245                 250                 255

Ile Ala Ala Leu Val Val Trp Ala Arg Asp Ile Pro His Phe Ser Gln
            260                 265                 270

Leu Glu Met Glu Asp Gln Ile Leu Leu Ile Lys Gly Ser Trp Asn Glu
        275                 280                 285

Leu Leu Leu Phe Ala Ile Ala Trp Arg Ser Met Glu Phe Leu Thr Glu
    290                 295                 300

Glu Arg Asp Gly Val Asp Gly Thr Gly Asn Arg Thr Thr Ser Pro Pro
305                 310                 315                 320

Gln Leu Met Cys Leu Met Pro Gly Met Thr Leu His Arg Asn Ser Ala
                325                 330                 335

Leu Gln Ala Gly Val Gly Gln Ile Phe Asp Arg Val Leu Ser Glu Leu
            340                 345                 350

Ser Leu Lys Met Arg Thr Leu Arg Val Asp Gln Ala Glu Tyr Val Ala
        355                 360                 365

Leu Lys Ala Ile Ile Leu Leu Asn Pro Asp Val Lys Gly Leu Lys Asn
    370                 375                 380

Arg Gln Glu Val Glu Val Leu Arg Glu Lys Met Phe Leu Cys Leu Asp
385                 390                 395                 400

Glu Tyr Cys Arg Arg Ser Arg Ser Ser Glu Glu Gly Arg Phe Ala Ala
                405                 410                 415

Leu Leu Leu Arg Leu Pro Ala Leu Arg Ser Ile Ser Leu Lys Ser Phe
            420                 425                 430

Glu His Leu Phe Phe Phe His Leu Val Ala Asp Thr Ser Ile Ala Gly
        435                 440                 445

Tyr Ile Arg Asp Ala Leu Arg Asn His Ala Pro Pro Ile Asp Thr Asn

    450                 455                 460
Met Met
465

## Beschreibung

Die Erfindung betrifft Nukleinsäuren, die für Polypeptide mit der biologischen Aktivität des Ultraspiracle-Proteins kodieren, sowie solche Polypeptide per se. Weiterhin betrifft die Erfindung Verfahren zum Auffinden insektizider Wirkstoffe, sowie zur kontrollierten Expression von Zielgenen (Gene Switch).

Das Ultraspiracle-Protein (im Folgenden als USP bezeichnet) ist das Insektenorthologe des Vertebraten Retinoid X Rezeptors (RXR). Wie RXR gehört es zur Familie der Kernrezeptoren. Diese Kernrezeptoren befinden sich im Zellinneren. Sie binden als Homo- oder Heterodimere an responsive Elemente auf der DNA und regulieren die Expression von Genen. Um aktiv zu sein, müssen sie spezielle kleine, hydrophobe Liganden (z.B. Steroide, Retinoide, Vitamin D) binden. Kernrezeptoren besitzen eine modulare Struktur mit funktionellen Domänen für Transaktivierung, DNA-Bindung und Ligandenbindung. Die DNA-Bindedomäne enthält eine Anzahl von Cystein-Resten und bildet eine charakteristische Struktur, den sogenannten Zinkfinger.

Aufgrund ihrer strukturellen und funktionellen Eigenschaften (DNA-Bindung an spezifische Elemente, Aktivierung nachgeschalteter Gene) eignen sich Kernrezeptoren als Komponenten für regulierbare Expressionssysteme (Gene Switch). Einige Kernrezeptoren (z.B. RXR, EcR) finden bereits Verwendung in induzierbaren, eukaryotischen Expressionssystemen (Invitrogen Corporation, Carlsbad CA, USA).

In Insekten wird z.B. die Entwicklung von der Larve zum adulten Insekt über Kernrezeptoren unter Beteiligung des Steroidhormons Ecdyson sowie des Isoprenoids Juvenilhormon gesteuert (1;2;3;4). Eine Schlüsselrolle spielt hierbei der Ecdysonrezeptor, ein Kernrezeptor, der aus zwei verschiedenen Untereinheiten, EcR und USP, zusammengesetzt ist (5;6;7). Während bereits seit langem das Hormon Ecdyson (in seiner aktiven Form 20-Hydroxyecdyson) als Ligand für die EcR-Untereinheit bekannt ist, handelt es sich bei USP um einen Orphanrezeptor, für den bisher noch kein Ligand identifiziert werden konnte.

Der Ecdysonrezeptor stellt ein wichtiges Insektizid-Target dar. Wird er außerhalb des in der Insektenentwicklung dafür vorgesehenen zeitlichen Fensters aktiviert, so führt dies zu schweren Störungen bis hin zum Absterben des Insekts. Auf diesem Mechanismus beruhen insektizide Ecdysonagonisten (8;9). Hierbei handelt es sich um nicht-steroidale Liganden der EcR-Untereinheit, die spezifisch auf Lepidopteren wirken (10). Da die Ecdyson/Juvenilhormon gesteuerte Entwicklung nur bei Invertebraten zu finden ist und in Vertebraten nicht vorkommt, stellt sie einen für den Anwender sicheren insektiziden Mechanismus dar.

Die Proteinsequenz einer Anzahl von Insekten-USPs ist bereits bekannt. So sind z.B. die Sequenzen von Drosophila melanogaster, Manduca sexta, Choristoneura fumiferana und Bombyx mori beschrieben (11).

Da es sich bei USP um einen Orphanrezeptor handelt, für den bisher kein Ligand bekannt ist, ist dieser Rezeptor von großer praktischer Bedeutung für die Etablierung von Screeningsystemen für die Suche nach neuen Liganden, die dann u.a. als Insektizide eingesetzt werden können. Stehen Liganden für USP bereit, so kann dieser Kernrezeptor in Systemen zur kontrollierten Expression von Zielgenen (Gene Switch) Verwendung finden.

Gegenstand der vorliegenden Erfindung sind Nukleinsäuren, welche für Polypeptide mit der biologischen Aktivität von USP kodieren und eine Sequenz umfassen, ausgewählt aus:
a) der Sequenz gemäß SEQ ID NO: 1,
b) Sequenzen, welche eine zumindest 85 %ige Identität, vorzugsweise eine zumindest 90%ige Identität, besonders bevorzugt eine zumindest 95%ige Identität mit der Sequenz gemäß SEQ ID NO: 1 über eine Länge von wenigstens 600 fortlaufenden Nukleotiden und vorzugsweise über deren Gesamtlänge aufweisen,
c) Sequenzen, welche aufgrund der Degeneration des genetischen Codes für dieselbe Aminosäuresequenz kodieren wie die unter (a) und (b) definierten Sequenzen,
d) Teile der unter (a), (b) und (c) definierten Sequenzen, die für Polypeptide kodieren, welche im Wesentlichen dieselbe biologische Aktivität ausüben wie ein Polypeptid mit der Aminosäuresequenz gemäß SEQ ID NO: 2.

Der Grad der Identität der Nukleinsäuresequenzen wird vorzugsweise bestimmt mit Hilfe des Programms GAP aus dem Programmpaket GCG, Version 9.1 unter Standardeinstellungen.

Gegenstand der Erfindung sind weiterhin Vektoren, die zumindest eine der erfindungsgemäßen Nukleinsäuren enthalten. Als Vektoren können alle in molekularbiologischen Laboratorien verwendete Plasmide, Phasmide, Cosmide, YACs oder künstliche Chromosomen verwendet werden. Für die Expression der erfindungsgemäßen Nukleinsäuren können diese mit üblichen regulatorischen Sequenzen verknüpft werden. Die Auswahl solcher regulatorischen Sequenzen ist davon abhängig, ob zur Expression prooder eukaryotische Zellen bzw. zellfreie Systeme verwendet werden. Besonders bevorzugt als Expressionskontrollsequenz sind z.B. der frühe oder späte Promotor des SV40 oder des Adenovirus, des Cytomegalovirus, der Immediate Early Promoter des AcMNPV, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase, der Promotor des α-Mating-Faktors der Hefe und der 35S Promoter des Blumenkohlmosaikvirus. Der Ausdruck "Promotor", wie er hierin verwendet wird, bezieht sich allgemein auf Expressionskontrollsequenzen.

Zur Expression der erfindungsgemäßen Nukleinsäuren können diese in geeignete Wirtszellen eingebracht werden. Der Ausdruck "Wirtszelle", wie er hierin verwendet wird, bezieht sich auf Zellen, die natürlicherweise die erfindungsgemäßen Nukleinsäuren nicht enthalten. Als Wirtszellen eignen sich sowohl prokaryotische Zellen, vorzugsweise E.coli, als auch eukaryotische Zellen wie Säuger- ,Insekten und Pflanzenzellen. Beispiele für geeignete einzellige Wirtszellen sind: Pseudomonas, Bacillus, Streptomyces, Hefen, HEK-293-, Schneider S2-, Sf9-, CHO-, COS1-, COS7-Zellen. Es eignen sich aber auch Zellen, die Bestandteil komplexer Systeme (z.B. ganze Pflanzen oder Tiere) sind. Daher sind auch transgene Organismen (ausgenommen Menschen), wie bespielsweise Pflanzen und Tiere, die die erfindungsgemäßen Nukleinsäuren enthalten, Gegenstand dieser Erfindung. Der Ausdruck "transgen", wie er hierin verwendet wird, bedeutet, dass die erfindungsgemäße Nukleinsäure mittels gentechnischer Verfahren in den Organismus eingebracht wurde.

Gegenstand der vorliegenden Erfindung sind auch die Polypeptide, die von den erfindungsgemäßen Nukleinsäuren kodiert werden, sowie die daraus aufgebauten Rezeptoren bestehend aus einer EcR-Untereinheit und einem erfindungsgemäßen Polypeptid.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die biologische Aktivität der erfindungsgemäßen Polypeptide lässt sich beispielsweise durch einen Transaktivierungstest nachweisen. Dazu wird ein zu testendes Polypeptid in Kombination mit einer EcR-Untereinheit und einem Reporterkonstrukt bestehend aus einem Promoter mit EcR-Bindungssequenz und einem Reportergen in einem Zellsystem exprimiert. Wenn in Anwesenheit von Ecdyson bzw. einer Ecdyson-analogen Verbindung das Reportergenprodukt z.B. durch einen Enzymtest detektiert werden kann, bedeutet dies, dass das getestete Polypeptid die biologische Aktivität eines erfindungsgemäßen Polypeptids besitzt.

Geeignete Reportergene und Bindungssequenzen sind beispielsweise in der WO 97/45737 beschrieben.

Die erfindungsgemäßen Polypeptide müssen nicht vollständige USPs darstellen, sondern können auch nur Fragmente davon sein, solange sie zumindest noch die biologische Aktivität eines Polypeptids (USP) mit der Aminosäuresequenz gemäß SEQ ID NO: 2 aufweisen. Dabei müssen die erfindungsgemäßen Polypeptide nicht direkt von einem USP von Heliothis virescens ableitbar sein.

Die erfindungsgemäßen Polypeptide können im Vergleich zu der entsprechenden Region eines natürlich vorkommenden USPs von Heliothis virescens Deletionen oder Aminosäuresubstitutionen aufweisen, solange sie zumindest noch die biologische Aktivität eines USPs ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Eine bevorzugte Ausführungsform der erfindungsgemäßen Polypeptide stellt ein USP von Heliothis virescens dar, welches die Aminosäuresequenz gemäß SEQ ID NO: 2 besitzt.

Weiterhin sind Antikörper Gegenstand der Erfindung, die spezifisch an die vorstehend genannten Polypeptide bzw. Rezeptoren binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin lässt sich in an sich bekannter Weise eine immortalisierte Zellinie erhalten, die monoklonale Antikörper produziert. Die Antikörper können gegebenenfalls mit einem Nachweisreagenz markiert sein. Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen. Daher erstreckt sich der Ausdruck "Antikörper", wie er hierin verwendet wird, auch auf Teile vollständiger Antikörper, wie Fa-, F(ab')₂- oder Fv-Fragmente, welche noch die Fähigkeit besitzen, an die Epitope der erfindungsgemäßen Polypeptide zu binden.

Zur Herstellung der Polypeptide, die von den erfindungsgemäßen Nukleinsäuren kodiert werden, können Wirtszellen, die zumindest eine der erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Die erfindungsgemäßen Nukleinsäuren können zur Isolierung und Charakterisierung der regulatorischen Regionen, die natürlicherweise benachbart zu der kodierenden Region vorkommen, verwendet werden. Solche regulatorischen Regionen sind somit ebenfalls Gegenstand der vorliegenden Erfindung.

Mit Hilfe der erfindungsgemäßen Nukleinsäuren können durch in vivo-Verfahren neue Liganden der USP-Untereinheit eines Ecdysonrezeptors identifiziert werden. Dazu kann beispielsweise ein rekombinantes DNA-Molekül, das zumindest eine erfindungsgemäße Nukleinsäure umfasst, in eine geeignete Wirtszelle eingebracht werden. Die Wirtzelle wird in Gegenwart einer chemischen Verbindung oder eines Gemisches an chemischen Verbindungen unter Bedingungen kultiviert, die die Expression der erfindungsgemäßen Polypeptide erlauben. Eine Aktivierung oder Hemmung des Rezeptors kann durch Transaktivierung eines Reportergens (z.B. Luciferase, beta-Galactosidase) detektierbar gemacht werden, dem ein geeigneter Promotor mit USP-Bindungssequenz (12) vorgeschaltet ist.

Die erfindungsgemäßen Nukleinsäuren ermöglichen auch das Auffinden von Verbindungen, die an die erfindungsgemäßen Polypeptide binden, durch in vitro-Verfahren. Die erfindungsgemäßen Polypeptide können mit einer chemischen Verbindung oder einem Gemisch von chemischen Verbindungen unter Bedingungen in Kontakt gebracht, die die Wechselwirkung zumindest einer Verbindung mit dem erfindungsgemäßen Polypeptid erlauben. Die Bindung von Verbindungen an ein erfindungsgemäßes Polypeptid kann z.B. durch Verdrängung eines radioaktiv- oder fluoreszenzmarkierten Liganden nachgewiesen werden. Hierzu kann auch ein erfindungsgemäßes Polypeptid markiert werden, um z.B. die Anwendung einer "Fluorescence Resonance Energy Transfer" (FRET)-Methode zu erlauben.

Auf diese Weise aufgefundene Liganden können als neue insektizide Substanzen im Pflanzenschutz Verwendung finden. Solche Liganden können kleine organischchemische Moleküle, Peptide oder Antikörper sein.

Eine weitere Anwendung der vorstehend beschriebenen erfindungsgemäßen Nukleinsäuren, Vektoren und regulatorischen Regionen besteht in ihrer Nutzung als chemisch induzierbare Expressionssysteme (Gene Switch) für unterschiedliche Zielgene. Hierzu können die Nukleinsäuren wie oben beschrieben in Wirtszellen exprimiert werden. Die Zielgene werden in Expressionsvektoren einkloniert, die mit einem geeigneten Promotor mit regulatorischen Regionen versehen sind. Diese Expressionsvektoren werden dann ebenfalls in die Wirtszellen gebracht. Die Regulierung der Zielgen-Transkription kann durch Zugabe eines wie oben beschriebenen Liganden zu den Wirtszellen erfolgen. Neben der Verwendung in kultivierten Zellen ist insbesondere die Verwendung in Pflanzen vorteilhaft, weil Pflanzen über keine endogenen Kernrezeptoren verfügen, und zur Zeit noch kein anderes gut funktionierendes chemisch induzierbares Expressionssystem für Pflanzen zur Verfügung steht. In der Erzeugung von Proteinen in Pflanzen liegt ein großes Potenzial. Aber auch therapeutische Anwendungen an Tieren einschließlich des Menschen sind möglich.

### Erläuterungen zum Sequenzprotokoll:

SEQ ID NO: 1 zeigt die Nukleotidsequenz des Heliothis virescens USP. SEQ ID NO: 2 zeigt die Aminosäuresequenz des von der Heliothis virescens USP-Nukleotidsequenz abgeleiteten Proteins.

### Beispiele:

### Beispiel 1

### Isolierung der beschriebenen Polynukleotide

Die Manipulation von Polynukleotiden erfolgte nach Standardmethoden der rekombinanten DNA Technologie (13). Die bioinformatische Bearbeitung von Nukleotid- und Aminosäuresequenzen erfolgten mit dem Programmpaket GCG Version 9.1 (GCG Genetics Computer Group, Inc., Madison Wisconsin, USA).

Die RNA für die cDNA-Bibliothek wurde aus gesamten Heliothis virescens-Larven (2. und 3. Larvenstadium) mittels Trizol-Reagenz (Gibco BRL, nach Angaben des Herstellers) isoliert. Aus diesen RNAs wurden nun die poly A enthaltenden RNAs durch Reinigung über Dyna Beads 280 (Dynal) isoliert. 5 µg dieser poly A enthaltenden RNAs wurden anschließend in die Konstruktion der cDNA-Bibliothek mit dem λ-ZAPExpress Vektor eingesetzt (cDNA Synthesis Kit, ZAP-cDNA Synthesis Kit und ZAP-cDNA Gigapack III Gold Cloning Kit, alle Stratagene). In Abweichung von den Angaben des Herstellers wurde zur cDNA-Synthese die Reverse Transkriptase Superscript (Gibco BRL) bei einer Synthesetemperatur von 45°C verwendet. Außerdem wurde auf die Zugabe radioaktiv markierter Desoxynukleosidtriphosphate verzichtet. Des Weiteren wurden die synthetisierten cDNAs nicht über das im Kit enthaltene Gelfiltrationsmedium, sondern über Size Sep 400 Spun Columns (Pharmacia) fraktioniert.

Alle Screens erfolgten mit Hilfe des DIG Systems (alle Reagenzien und Verbrauchsmaterialien Boehringer Mannheim, nach Angaben im "The DIG System User's Guide for Filter Hybridization", Boehringer Mannheim). Die eingesetzten DNA-Sonden wurden durch PCR mittels Digoxygenin markiertem dUTP präpariert. Die Hybridisierungen erfolgten in DIG Easy Hyb (Boehringer Mannheim) bei 40°C über Nacht. Der Nachweis markierter DNA auf Nylonmembranen geschah durch Chemolumineszenz (CDP-Star, Boehringer Mannheim) unter Verwendung von Röntgenfilmen (Lumifilm, Fa. Boehringer Mannheim). Die isolierten Genbankplasmide wurden zur Identifikation mittels T3 und T7 Primer ansequenziert (ABI Prism Dye Terminator Cycle Sequencing Kit, ABI, mit ABI Prism 310 Genetic Analyzer). Die Bestimmung der vollständigen Polynukleotidsequenzen erfolgte durch Primer Walking mittels Cycle Sequencing als Auftragssequenzierung bei der Firma MediGene, Martinsried.

### Literatur:

1. Segraves W.A. (1994): Steroid Receptors and Other Transcription Factors in Ecdysone Response. Recent Progress in Hormone Research, 49, 167-195
2. Henrich V.C. & Brown N.E. (1995): Insect Nuclear Receptors: A Developmental and Comparative Perspective. Insect Biochem. Mol. Biol. 25 (8), 881-897
3. Thummel C.S. (1995): From Embryogenesis to Metamorphosis: The Regulation and Function of Drosophila Nuclear Receptor Superfamily Members. Cell 83, 871-877
4. Truman J.W. (1996): Ecdysis Control Sheds Another Layer. Science 271, 40-41
5. Yao T et al. (1993): Functional ecdysone receptor is the product of EcR and Ultraspiracle genes. Nature 366, 476-479
6. Hall B.L. & Thummel C.S. (1998): The RXR homolog Ultraspiracle is an essential component of the Drosophila ecdysone receptor. Development 125, 4709-4717
7. Lezzi M. et al. (1999): The Ecdysone Receptor Puzzle. Arch. Insect Biochem. Physiol. 41, 99-106
8. Mikitani K. (1996): Ecdysteroid Receptor Binding Activity and Ecdysteroid Agonist Activity at the Level of Gene Expression are Correlated with the Activity of Dibenzoyl Hydrazines in Larvae of Bombyx mori. J. Insect Physiol. 42 (10), 937-941
9. Dhadialla T.S. et al. (1998): New Insecticides with Ecdysteroidal and Juvenile Hormone Activity. Annu. Rev. Entomol. 43, 545-569
10. Sundaram M. et al. (1998): Basis for selective action of a synthetic molting hormone agonist, RH-5992 on lepidopteran insects. Insect Biochem. Mol. Biol. 28, 693-704
11. Oro A.E. et al. (1990): Relationship between the product of the Drosophila ultraspiracle locus and the vertebrate retinoid X receptor. Nature 347, 298-301
12. Vögtli M. et al. (1998): High level transactivation by the ecdysone receptor complex at the core recognition motif. Nucl. Acid Res. 26 (10), 2407-2414
13. Sambrook et al. (1989): Molecular Cloning, A Laboratory Manual, 2nd ed. Cold Spring Harbor Press

## Patentansprüche

1. Nukleinsäure, die für ein Polypeptid mit der biologischen Aktivität des Ultraspiracle-Proteins kodiert, umfassend eine Sequenz ausgewählt aus
(a) der Sequenz gemäß SEQ ID NO: 1,
(b) Sequenzen, welche eine zumindest 85%ige Identität mit der Sequenz gemäß SEQ ID NO: 1 über eine Länge von wenigstens 600 fortlaufenden Nukleotiden aufweisen,
(c) Sequenzen, welche aufgrund der Degeneration des genetischen Codes für dieselbe Aminosäuresequenz kodieren wie die unter (a) und (b) definierten Sequenzen,
(d) Teile der unter (a), (b) und (c) definierten Sequenzen, die für Polypeptide kodieren, welche im Wesentlichen dieselbe biologische Aktivität ausüben wie ein Polypeptid mit der Aminosäuresequenz gemäß SEQ ID NO: 2.

2. Vektor umfassend zumindest eine Nukleinsäure gemäß Anspruch 1.

3. Vektor nach Anspruch 2, **dadurch gekennzeichnet, dass** das Nukleinsäuremolekül funktionell mit regulatorischen Sequenzen verknüpft ist, die die Expression der Nukleinsäure in pro- oder eukaryotischen Zellen gewährleisten.

4. Wirtszelle enthaltend eine Nukleinsäure gemäß Anspruch 1 oder einen Vektor gemäß Anspruch 2 oder 3.

5. Wirtszelle nach Anspruch 4, **dadurch gekennzeichnet, dass** es sich um eine pro- oder eukaryotische Zelle handelt.

6. Wirtszelle nach Anspruch 5, **dadurch gekennzeichnet, dass** die prokaryotische Zelle E.coli ist.

7. Wirtszelle nach Anspruch 5, **dadurch gekennzeichnet, dass** die eukaryotische Zelle eine Hefe-, Säuger-, Insekten- oder Pflanzenzelle ist.

8. Transgener Organismus, ausgenommen der Mensch, enthaltend eine Nukleinsäure gemäß Anspruch 1 oder einen Vektor gemäß Anspruch 2 oder 3.

9. Polypeptid, welches von einer Nukleinsäure gemäß Anspruch 1 kodiert wird.

10. Rezeptor umfassend eine EcR-Untereinheit und ein Polypeptid gemäß Anspruch 9.

11. Antikörper, welcher spezifisch an ein Polypeptid gemäß Anspruch 9 bindet.

12. Verfahren zur Herstellung eines Polypeptids gemäß Anspruch 9 umfassend die folgenden Schritte:
(a) Kultivieren einer Wirtszelle gemäß einem der Ansprüche 4 bis 7 unter Bedingungen, die die Expression der Nukleinsäure gemäß Anspruch 1 gewährleisten, und
(b) Gewinnen des Polypeptids aus den Zellen oder dem Kulturmedium.

13. Verfahren zur Herstellung einer Nukleinsäure gemäß Anspruch 1 umfassend die folgenden Schritte:
(a) Vollständige chemische Synthese auf an sich bekannte Weise oder
(b) chemische Synthese von Oligonukleotiden, Markieren der Oligonukleotide, Hybridisieren der Oligonukleotide an DNA einer Insekten-cDNA-Bank, Selektieren von positiven Klonen und Isolieren der hybridisierenden DNA aus positiven Klonen oder
(c) chemische Synthese von Oligonukleotiden und Amplifizierung der Ziel-DNA mittels PCR.

14. Regulatorische Region, welche natürlicherweise die Transkription einer Nukleinsäure gemäß Anspruch 1 in Insektenzellen kontrolliert und eine spezifische Expression gewährleistet.

15. Verfahren zum Auffinden neuer Wirkstoffe für den Pflanzenschutz, insbesondere von Verbindungen, welche die Aktivierung oder Hemmung eines Polypeptids gemäß Anspruch 9 oder eines Rezeptors gemäß Anspruch 10 verursachen, umfassend die folgenden Schritte:
(a) Bereitstellen einer Wirtszelle gemäß einem der Ansprüche 4 bis 7,
(b) Kultivieren der Wirtszelle in der Gegenwart einer chemischen Verbindung oder eines Gemischs von chemischen Verbindung, und
(c) Detektieren der Aktivierung bzw. der Hemmung des Polypeptids bzw. Rezeptors.

16. Verfahren zum Auffinden einer Verbindung, die an ein Polypeptid gemäß Anspruch 9 bindet, umfassend die folgenden Schritte:
(a) Inkontaktbringen eines Polypeptids gemäß Anspruch 9 mit einer Verbindung oder einem Gemisch von Verbindungen unter Bedingungen, die die Interaktion der Verbindung(en) mit dem Polypeptid erlauben, und
(b) Bestimmen der Verbindung, die spezifisch an das Polypeptid bindet.

17. Verfahren zur induzierbaren Expression von Zielgenen mittels eines Polypeptids gemäß Anspruch 9, umfassend die folgenden Schritte:
(a) Kultivieren einer Wirtszelle gemäß einem der Ansprüche 4 bis 7 oder Bereitstellen eines transgenen Organismus gemäß Anspruch 8 unter Bedingungen, die die Expression der Nukleinsäure gemäß Anspruch 1 gewährleisten, wobei die Wirtszelle bzw. der transgene Organismus ein Zielgen mit geeigneten regulatorischen Sequenzen enthält, und
(b) Inkontaktbringen der Wirtszelle bzw. des transgenen Organismus mit einer chemischen Verbindung, die die Expression des Zielgens induziert.

18. Verwendung zumindest einer Nukleinsäure gemäß Anspruch 1, eines Vektors gemäß Anspruch 2 oder 3, einer Wirtzelle gemäß einem der Ansprüche 4 bis 7, eines transgenen Organismus gemäß Anspruch 8, eines Polypeptids gemäß Anspruch 9, eines Rezeptors gemäß Anspruch 10 oder einer regulatorischen Region gemäß Anspruch 14 zum Auffinden neuer Wirkstoffe für den Pflanzenschutz.

19. Verwendung zumindest einer Nukleinsäure gemäß Anspruch 1, eines Vektors gemäß Anspruch 2 oder 3, einer Wirtzelle gemäß einem der Ansprüche 4 bis 7, eines transgenen Organismus gemäß Anspruch 8, eines Polypeptids gemäß Anspruch 9, eines Rezeptors gemäß Anspruch 10, einer regulatorischen Region gemäß Anspruch 14 oder eines Verfahrens gemäß Anspruch 17 zur gezielten Veränderung der biologischen Eigenschaften einer Wirtszelle oder eines Wirtsorganismus.
